# EUROPEAN PATENT APPLICATION

(11) **EP 2 189 168 A1**
(43) Date of publication of application: **26.05.2010**
(21) Application number: 09180581.2
(22) Date of filing: 01.12.2006
(51) Int. Cl.: A61L 27/20, A61L 27/54, A61L 31/04, A61L 31/16, A61F 2/02, A61K 31/717

(54) **Implantable microbial cellulose materials for various medical applications**

(30) Priority: 02.12.2005 US 292075
(62) Divisional of application: 06024913.3
(71) Applicant: Xylos Corporation, Langhorne, PA 19047 (US)
(72) Inventor: Serafica, Gonzalo, Langhorne, PA 19047 (US); Damien, Chris, Newton, PA 18940 (US); Wright, Frederic, S., Ardmore, PA 19003-1815 (US); Beam, Heather, Tinton Falls, NJ 07712 (US)
(74) Representative: HOFFMANN EITLE

(57) **Abstract**

This invention relates to polysaccharide materials, especially microbial cellulose processed by drying and then compressing to obtain suitable implantation properties for repair or replacement of soft tissue. The invention relates to the use of the implantable microbial cellulose for tissue closure reinforcement, buttresses for reinforcement of the soft tissue, rotator cuff repair, and as carrier vehicles for medically useful substances for repair or regeneration of tissue.

## Description

### BACKGROUND OF THE INVENTION

### Field of Invention

This invention relates to polysaccharide materials and more particularly to microbial cellulose having suitable implantation properties for repair or replacement of soft tissue. The invention also relates to the use of the implantable microbial cellulose as, tissue closure reinforcement, buttresses s for reinforcement of the soft tissue, adhesion barriers, articular cartilage repair, pericardial patches, and as carrier vehicles for drug or other active agent delivery for repair or regeneration of tissue.

### Description of the related art

Various materials used as implantable devices in the medical industry have been well documented and can be divided into biologic, synthetic and biosynthesized. Biologic materials include autograft tissue (a patient's own tissue), allograft (tissue from another individual of the same species) and xenograft (tissue from another species). While autograft often remains the gold standard, the harvest of tissue from one part of a body to be implanted into another part carries a degree of morbidity; often the harvest site being more painful than the implant site. Allograft, as described in U.S. Patent Nos. 5,072,373; 5,290,558; 5,510,396; 6,030,635; and 6, 785, 863, has been used as a medical implant for a variety of indications including as a bone graft substitute and in the repair of rotator cuff defects. Xenograft, including collagen, has been implanted as bone graft substitutes (U.S. Patent No. 5,830,493), tendon and ligament repair, surgical staple buttressing (U.S. Patent No. 5,810,855) and other tissue repair and replacement (U.S. Patent Nos. 6,179,872 and 6,206,931). These tissues carry the risk of disease transmission from donor to host and are often cross-linked, using cytotoxic chemicals, to improve their mechanical strength and degradation profile.

Synthetic materials include polymers comprising of polylactic (PLA), polyglycolic acid (PGA) and polypropylene, which have long been used as surgical sutures. These synthetic materials have been fabricated into films, mesh and more complex three dimensional structures depending on intended applications as described in U.S. Patent Nos. 5,441,508; 5,830,493; 6,031,148; 6,852,330; 5,946,003; and Koh J.L., et al. Supplementation of Rotator Cuff Repair with a Bioresorbable Scaffold, Am. J. Sports Med. 30:410-413, 2002. U.S. Patent Nos. 6,156,056; 6,245,081; 6,620,166; and 6,814,741 describe the use of polymer based suture buttresses that anchor into a bone tunnel and then attach to a suture. Another example of a widely used synthetic material is poly(tetrafluoroethylene) PTFE, which has been used in wide array of medical implantable articles including vascular grafts (U.S. Patent Nos. 4,946,377 and 5,718,973), tissue repair sheets and patches (U.S. Patent No. 5,433,996). The PTFF material has also been used as a surgical staple line reinforcement device as described in U.S. Patent 5,702,409 and 5, 810, 855. Polymeric hydrogels have also been adapted for surgical implants (U.S. Patent No. 4,836,884); finding uses such as soft tissue and blood vessel substitutes.

These synthetic materials possess certain physical characteristics that make them suitable as an implant material. Such properties include biocompatibility, strength, chemically stability, etc. which can be particularly important for a specific application. For example, PTFE has the strength and interconnecting fibril structure that is critical in fabrication of tubular grafts. Synthetic hydrogels, which have a superficial resemblance to living tissue due to high water content, display minimal irritation to surrounding tissues making them useful as prosthetic devices. However, these synthetic materials also have limitations and disadvantages such as a limited range of physical and biochemical properties, unfavorable degradation products and profiles, leaching of chemicals, and difficult handling properties. Thus, there remains a need to explore alternative materials more suitable for specific surgical applications.

Biosynthetic materials have also been used for tissue repair and augmentation. Chitosan, dextran and polyhydroxyalkanoate (PHA) polymers (U.S. Patent No. 6,867,247) can all be considered biosynthetic to mean produced by living organism. Chitosan is produced by certain shellfish, while dextran and PHA have been synthesized from bacteria. These materials have been suggested for use in various medical implantable applications that include tissue repair patches, tacks and sutures, as well scaffolds for soft tissue regeneration. Other applications include the use of these materials as skin substitutes, wound dressing and hemostatic agent. Another biomaterial that has had extensive use for surgical applications is cellulose and the use of viscose or regenerated cellulose as implantable articles is known. Several investigators have studied tissue biocompatibility of cellulose and its derivatives (Miyamoto, T. et al., Tissue Biocompatibility of Cellulose and its derivatives. J. Biomed. Mat. Res., V. 23, 125-133 (1989)) as well as examined some specific applications for the material. The oxidized form of regenerated cellulose has long been used as a hemostatic agent and adhesion barrier (Dimitrijevich, S. D., et al. In viva Degradation of Oxidized regenerated Cellulose. Carbohydrate Research, V. 198, 331-341 (1990), Dimitrijevich, S. D., et al. Biodegradation of Oxidized regenerated Cellulose Carbohydrate Research, V. 195, 247-256 (1990)) and are known to degrade much faster than the non-oxidized counterpart. A cellulose sponge studied by Martson, et al., showed excellent biocompatibility with bone and connective tissue formation during subcutaneous implantation (Martson, M., et al., Is Cellulose sponge degradable or stable as an implantation material? An in vivo subcutaneous study in rat. Biomaterials, V. 20, 1989-1995 (1999), Martson, M., et al., Connective Tissue formation in Subcutaneous Cellulose sponge Implants in rats. Eur. Surg. Res., V. 30, 419-425 (1998), Martson, M., et al., Biocompatibility of Cellulose Sponge with Bone. Eur. Surg. Res., V. 30, 426-432 (1998)). The authors surmised that cellulose material can be a viable long term stable implant. Other forms and derivatives of cellulose have also been investigated (Pajulo, O. et al. Viscose cellulose Sponge as an Implantable matrix: Changes in the structure increase production of granulation tissue. J. Biomed. Mat. Res., V. 32, 439-446 (1996), Mello, L. R., et al., Duraplasty with Biosynthetic Cellulose: An Experimental Study. Journal of Neurosurgery, V. 86, 143-150 (1997)).

However, the prior art mentions only limited applications of microbial cellulose. For example, the use of microbial cellulose in the medical industry has beer described for liquid loaded pads (U.S. Patent No. 4,588,400), skin graft or vulnerary covers (U.S. Patent 5,558,861), wound dressings (U.S. Patent No. 5,846,213) and topical applications (U.S. Patent No. 4,912,049). These patents have focused on the use of microbial cellulose for topical applications and have not cited its particular application as implantable materials. Mello et al. described above suggests the use of microbial cellulose in duraplasty, but describes a stretch drying method that does not produce a mechanically strong material. The only patent that describes the use of microbial cellulose obtained from *Acetobacter xylinum* as an implant is U.S. Patent No. 6,599,518 wherein a solvent dehydrated microbially derived cellulose material can be used specifically for tissue repair materials, tissue substitutes and bulking agents for plastic and reconstructive. The materials described by the '518 patent differ from the instant invention in that they possess physical characteristics such as minimal elongation, high rigidity and inability to absorb considerable amounts of fluid. These attributes render the implant material non-conformable and therefore not useful for particular surgical applications such as soft tissue augmentation or buttressing and musculoskeletal tissue reinforcement, repair, or replacement. Also, the '518 patent does not specify other processing methods other than solvent dehydration at ambient pressure to produce implantable products. The salt remaining from the process and the solvent drying at ambient pressure serve to stiffen the material. The form of the material in '518 allows for only minimal absorption of liquid and therefore only minimal swelling while having high tensile strength. Thus, the materials in '518 have limited fluid absorption capability and are not capable of being loaded with liquid solutions limiting its ability to deliver liquids containing drugs, biologics and other bioactive agents

The present invention describes a novel combination of drying and compressing to obtain strong, yet more conformable implant materials. The instant invention can have a varied pore size depending on the amount of compression and can therefore absorb liquid, swell to fill a space or have minimal swelling, yet increased conformability over the '518 material.

Another potential application is the use of the absorptive properties of the processed microbial cellulose to load a liquid soluble medicament inta the microbial cellulose for use as an anti-infective or drug delivery system.

Potential applications of the microbial cellulose have not been mentioned in the '518 patent. For example, the used of microbial cellulose as a buttress material to reinforce tissue during rotator cuff surgery has not been previously disclosed. Other specific applications of the material in this patent including adhesion barriers, articular cartilage repair, and pericardial patches are also described.

Accordingly, heretofore there has not been provided an acceptable implantable material comprising microbial cellulose for use in soft tissue repair, regeneration or replacement applications. Accordingly, there remains a need for an implantable material comprising microbial cellulose that is processed differently from previously described materials. This novel processing results in an implantable material with desirable properties and that can be used in a variety of surgical and drug delivery applications. Methods of implanting microbial cellulose such as open, laparoscopic, arthroscopic, endoscopic or percutaneous methods are also particularly desirable and attainable with more conformable microbial cellulose.

### SUMMARY OF THE INVENTION

The invention provides a new microbial cellulose that has at the same time good mechanical properties and good absorption properties. This material is thus particularly suitable as implant material. The tensile strength of the material is preferably at least 80 N, and its absorption capability is preferably at least 3 g liquid/g cellulose, more preferably at least 3.2 g liquid/g cellulose.

The invention also provides a new process for preparing microbial cellulose, comprising the steps of drying microbial cellulose by supercritical fluid drying and subsequently pressing the dried microbial cellulose.

The material can be used for providing a tissue scaffold, a suture buttress composition, or a shoulder repair composition.

The invention also provides an implantable composition comprising microbial cellulose and a medically useful substance.

There is provided, in accordance with one preferred embodiment of the invention, a new class of implantable materials utilizing microbial cellulose for medical and surgical applications of soft tissue repair and reinforcement (staple, suture, etc.) including rotator cuff repair.

There is provided, in accordance with another preferred embodiment of the invention, methods of implanting microbial cellulose in a wide variety of applications that utilize the desirable physical and chemical properties of microbial cellulose.

There is provided, in accordance with another preferred embodiment of the invention, a process for the preparation of these aforementioned materials that will yield the desirable properties for particular product applications.

There is provided, in accordance with another preferred embodiment of the invention, a process for the preparation of these aforementioned materials whereby a liquid medicament is absorbed (loaded) into the material and that yields the desirable properties for particular product applications.

### DESCRIPTION OF FIGURES

Figure 1 is a graph of the mechanical properties of three variations of implantable microbial cellulose illustrating the effect of cellulose content on the mechanical properties of tensile strength, elongation, suture retention strength and Young's Modulus.
Figure 2 is a graph of the mechanical properties comparing control and compressed implantable microbial cellulose illustrating the effect of processing and cellulose content on the mechanical properties of tensile strength, elongation, suture retention strength and stiffness.
Figure 3 is an SEM image taken in the horizontal plane of implantable microbial cellulose after supercritical fluid drying.
Figure 4 is an SEM image taken in the vertical plane of implantable microbial cellulose after supercritical drying.
Figure 5 is a graph of the tensile strength versus absorption amount comparing material from the '518 patent to those of the instant invention.
Figure 6 is a graph of the absorption over time of the materials of the instant invention compared to that of material prepared using the method of '518.

### DETAILED DESCRIPTION OF THE PREFERRED EMBODIMENTS

The present invention describes an implantable material comprising microbial cellulose. The instant implantable material has those properties necessary for in viva applications, for example, the implantable material of the instant invention can be adapted to a three-dimensional shape and possess desired fluid absorption and pliability characteristics.

The implantable materials of the instant invention are comprised of microbial cellulose. Those methods of preparing microbial cellulose are known to those of ordinary skill and are described, for example, in U.S. Patent Nos. 5,846,213 and 4,912,049, which are incorporated herein by reference in their entirety. Any cellulose producing organism can be used in producing the raw biosynthetic cellulose material. However, biosynthetic cellulose produced from a static culture of *Acetobacter xylinum* is preferred.

The microbial cellulose content of the raw material is dependent on the amount of media supplied to the *A.x*. bacteria. Once the pellicle is harvested, the raw material is physically and chemically processed so as to be a suitable implantable material for medical and surgical uses. For example, the microbial cellulose is first processed and cleaned to remove all non-cellulose material embedded in the cellulose pad and then depyrogenated using chemicals such as sodium hydroxide. After depyrogenation, the cellulose may be cross-linked by irradiation or chemical means if its strength needs to be adjusted. Addition of other agents, such as glycerol and polyethylene glycol used to modify the cellulose surface can also be performed in order to control water absorption and pliability which are desirable properties for implantable materials. The material can remain wet, moist, partially dehydrated, or totally dehydrated by air, heat, lyophilization, freeze-drying or supercritical fluid drying. In a preferred embodiment, the microbial cellulose is dried, drying being a removal of liquid, which is different from the dehydration described in US 6,599,518 B2.

Supercritical fluid drying is preferred. Supercritical fluid drying refers to a drying step using a fluid held at a pressure and temperature above its critical point. The critical point is the lowest pressure and temperature at which the fluid can co-exist as a gas and a liquid. For example, the critical pressure and temperature of carbon dioxide are 72.8 atmospheres and 31.1°C, respectively. Carbon dioxide held at a pressure and temperature above its critical point is in a supercritical condition or state. According to the present invention, carbon dioxide is preferred as a fluid. The term "supercritical drying" thus refers to a process that uses one or more fluids, with at least one being under supercritical conditions part of the time, to displace and remove water within the matrix of microbial cellulose. It is generally desirable that the pressure of the supercritical fluid be reduced over a period of time of from about 1 minute to about 120 minutes to prevent rapid cooling which causes condensation. Supercritical drying of microbial cellulose is described in US 5,772,647 and US 5,580,348. The methods described in these patents can also be used according to the present invention.

The material may be further processed by physically modifying the cellulose, preferably by compressing to a thin film after drying by applying repeated or sustained force directly to the dried material.

In a preferred embodiment, the pressing step is achieved by constant pressure. Alternatively, the pressing can be achieved by repeated hammering, and the material further sterilized for applications as medical implantable articles using standard sterilization methods such as gamma irradiation, e-beam irradiation, ethylene oxide or steam sterilization.

The combination of supercritical drying and subsequent compressing provides a material that is particularly suitable for implant materials. Preferably, the microbial cellulose content is 1 mg/cm² to 50 mg/cm². The tensile strength is preferably at least 80 N, more preferably in the range of 90 to 200 N. The absorption capability is preferably at least 3 g, more preferably at least 3.2 g, most preferably in the range 4.5 to 10 g, per gram of cellulose. The measurement methods for determining the tensile strength and the absorption capability are described below. Moreover, the microbial cellulose of the present invention has preferably a mean pore size of at least about 0.01, more preferably in the range of 0.1 to 500 µm. Moreover, its bulk density is preferably in the range of 0.0001 to about 0.5 g/cm³. The pore size and the bulk density are determined as described in US 5,580,348 (see column 9, lines 6 to 63).

Subsequent to the drying and physical modification step, the dried cellulose may be re-hydrated.

In one preferred embodiment, the invention provides a method for preparing an implantable device for medical and surgical applications comprising the steps of providing a microbial cellulose material; and incorporating said material into an implantable device for medical and surgical applications. Once produced, the microbial cellulose may be incorporated or fashioned into medical devices by commonly known methods such as molding, cross-linking, chemical surface reaction, dehydrating and/or drying, cutting or punching. Such medical devices include tissue substitutes or scaffolds for repair or reinforcement of damaged soft tissue.

Physical properties of microbial cellulose such as tensile strength, three dimensional structure, suture retention, absorption and conformability may be measured to show its characteristics by commonly utilized techniques such as scanning electron microscopy (SEM), mechanical testing or other standard physical tests. Chemical properties such as degree of crystallinity, active chemical groups and degree of polymerization can also be examined by techniques such as x-ray crystallography. Finally, the biocompatibility/safety properties of the Implantable microbial cellulose *in vitro* and in vivo may be assessed.

The properties of implantable microbial cellulose may be compared to a wide variety of implantable materials available including polypropylene mesh, PTFE, polymeric hydrogels, collagen, and human or animal derived tissue currently being used in the medical industry. Based on the results of these comparisons, including strength, conformability, and adhesion properties, a number of Implantable microbial cellulose articles may be tailored for specific applications.

The instant microbial cellulose may be used as a substitute or scaffold in tissue engineering. In this embodiment, the cellulose acts as a scaffold or trellis on which new tissue forms, orients and matures.

In a preferred embodiment, the invention provides a method of tissue reinforcement, comprising an Implantable composition comprising microbial cellulose and implanting said composition into a subject in need thereof. For example, the instant invention may be easily prepared as a dry or hydrated pad for direct application on a tissue through which staples, sutures or bone anchors are being added to ensure attachment of the tissue to its supporting structure. Often the tissue that is being repaired is friable and sutures or staples alone result in cutting or tearing and re-opening of the wound. In this embodiment the staples and/or sutures pass through both cellulose and tissue, the cellulose acting to reinforce the tissue by creating a stronger backing for attachment. For this application the material must be conformable so as to not cause damage to the tissue by rubbing, sharp edges, etc.

The device may be a tissue scaffold, a surgical suture reinforcement device, a surgical staple reinforcement device, an adhesion barrier, a device used in shoulder repair, preferably shoulder repair involving the rotator cuff or involving the labrum, or a suture repair preferably including anchoring tissue to the bone. The device can be implanted through an open procedure or through an arthroscope.

The invention also provides a kit comprised of a microbial cellulose device according to the present invention and a sterilizable closable container. The closable container may be a sealable pouch or a thermoformed tray with lid.

In a preferred embodiment, the invention provides a method for repair of the rotator cuff and other shoulder related tears using the cellulose material. A method or process for fabricating such implantable materials will be cited in the examples accordingly.

The material can be used for reinforcing tissue in and around the shoulder. The microbial cellulose described can be processed using the methods described above to create a sheet with multi-directional strength that can be used as a surgical device for rotator cuff repair. This may include both open and arthroscopic repair and include suture or staple reinforcement.

The instant invention also contemplates an implantable composition comprising microbial cellulose and a medically useful agent dissolved in the liquid that is absorbed by the microbial cellulose material. The implant material is then able to have a dual function such as structural support to the body and as a reservoir for bioactive agents or drug for delivery to the site of implantation. Alternatively the agent may be incorporated into the microbial cellulose to reduce the risk of infection or rejection of the implant. Any number of medically useful agents for tissue repair can be used in the invention by adding the substances to an implantable composition comprising the microbial cellulose carrier, either at any steps in the manufacturing process or directly to the final composition.

A medically useful agent is one having therapeutic, healing, curative, restorative, or medicinal properties. Such medically useful agents include collagen and insoluble collagen derivatives and soluble solids and/cr liquids dissolved therein. Also included are amino acids, peptides, vitamins, co-factors for protein synthesis; hormones; endocrine tissue or tissue fragments; synthesizers; enzymes such as collagenase, peptidases, oxidases; cell scaffolds with parenchymal cells; angiogenic drugs and polymeric carriers containing such drugs; collagen lattices; biocompatible surface active agents, antigenic agents; cytoskeletal agents; cartilage fragments, living cells such as chondrocytes, bone marrow cells, mesenchymal stem cells, natural extracts, tissue transplants, bioadhesives, transforming growth factor (TGF-beta) and associated family proteins (bone morphogenetic protein (BMP), growth and differentiation factors (GDF) etc.), fibroblast growth factor (FGF), insulin-like growth factor (IGF-1) and other growth factors; growth hormones such as somatotropin; bone digestors; antitumor agents; fibronectin; cellular attractants and attachment agents; immuno-suppressants; permeation enhancers; and peptides, such as growth releasing factor, P-15 and the like.

The drug can be in its free base or acid form, or in the form of salts, esters, or any other pharmacologically acceptable derivatives, enantomerically pure forms, tautomers or as components of molecular complexes. The amount of drug to be incorporated in the composition varies depending on the particular drug, the desired therapeutic effect, and the time span for which the device is to provide therapy. Generally, for purposes of the invention, the amount of drug in the system can vary from about 0.0001% to as much as 60%, preferably from about 0.01 wt.-% to about 50 wt.-%, more preferably from about 0.1 wt.-% to 10 wt.-%, with respect to the total weight of the composition

The active agent may be used to reduce inflammation, increase cell attachment, recruit cells, and/or cause differentiation of the cells to repair the damaged tissue.

In addition, implantable materials using microbial cellulose may be applied in a number of other useful areas, including, but not limited to other soft tissue substitutes or scaffolds.

Other objects, features and advantages of the present invention will become apparent from the following examples. It should be understood, however, that the detailed description and the specific examples, while indicating preferred embodiments of the invention, are given by way of illustration only, since various changes and modifications within the spirit and scope of the invention will become apparent to those skilled in the art from this detailed description. The invention, thus generally described, will be understood more readily by reference to the following examples, which are provided by way of illustration and are not intended to be limiting of the present invention.

### Example 1:

### Implantable Cellulose Preparation

To prepare the microbial cellulose of the invention, *Acetobacter xylinum* microorganisms were culture in a bioreactor containing a liquid nutrient medium at 30 degrees Celsius at an initial pH of 3-6. The medium was based on sucrose or other carbohydrates.

The bioreactor was composed of a plastic box fitted with an airtight cover. Dimensions of the bioreactor measured 3.5 in x 3.5 in. An aeration port was made in the bioreactor that allowed the proper oxygen tension to be achieved.

The fermentation process under static conditions was allowed to progress for a period of about 10-14 days, during which the bacteria in the culture medium produced an intact cellulose pellicle. Once the media was expended, the fermentation was stopped and the pellicle removed from the bioreactor. This material was termed '250'.

### 1. Processing and Depyrogenation Procedures

The excess medium contained in the pellicle was removed by mechanical compression prior to chemical cleaning and subsequent processing of the pellicle. The cellulose pellicle was subjected to a series of chemical wash steps to convert the raw cellulose film into a medical grade and nonpyrogenic implantable material. Processing started with an 8% sodium hydroxide solution at 70-75 degrees Celsius for 1 hour, followed by a rinse in deionized water and then a soak in 0.25% hydrogen peroxide at 70-75 degrees Celsius for 1 hour.

The resulting films were tested for pyrogens and mechanical properties. The amount of cellular debris left in the cellulose pad after processing is measured by validated Limulus Amoebocyte Lysate (LAL) testing as outlined by the U.S. Food and Drug Administration (FDA) in 21 CFR10.90. The instant cleaning process outlined above provided a nonpyrogenic cellulose pad (≤0.50 EU/ml). The steps of the LAL test are defined by the test kit manufacturer and can simply be followed to yield the pyrogen level in the cellulose film.

### 2. Final product processing

Once cleaned, the pellicles were mechanically pressed to reduce the water content. The materials were then soaked in 100% methanol for approximately 1 hour. The methanol water mixture was decanted and the samples soaked again in 100% methanol overnight. The methanol was changed at approximately 16 hours and again at 24 hours. Following the methanol exchange, the pellicle was repressed to reduce the methanol. Pellicles were then placed into a pressure vessel separated by polypropylene mesh and underwent supercritical carbon dioxide drying (SCD) at 2000 psi and 40 degrees Celsius until the methanol was removed and the material dry.

The resulting dry pad is cut to shape, packaged in single or dual foil pouches and sterilized by gamma irradiation at 25-35 kGy.

### Example 2:

Material was prepared the same as in Example 1, however additional media was added at the start and the pellicle was allowed to grow 14-17 days. The resulting pellicle was termed '360'. The cleaning, whitening, drying, packaging and sterilization were identical to Example 1.

### Example 3:

Material was prepared the same as in Example 1, however additional media was added at the start and the pellicle was allowed to grow 21-25 days. The resulting pellicle was termed '440'. The cleaning, whitening, drying, packaging and sterilization were identical to Example 1.

### Example 4:

Material was initially cleaned, whitened, and dried as in Example 2. It was further processed by subjecting it to mechanical pressure to create a thinner version termed '360P'. For this example the material was hammered with a plastic mallet to compress the cellulose into a thin wafer. This was then packaged and sterilized as in Example 1.

### Example 5:

Material was initially cleaned, whitened, and dried as in Example 3. It was further processed by subjecting it to mechanical pressure to create a thin version termed '440P'. For this example the material was hammered with a plastic mallet to compress the cellulose into a thin wafer. This was then packaged and sterilised as in Example 1.

### Example 6:

### Mechanical properties of Prepared Implantable Microbial Cellulose materials

Materials were processed as in Examples 1 - 3. The mechanical properties of these implantable microbial cellulose forms were analyzed using a United Tensile Tester (Model SSTM-2kN), including tensile strength, elongation, and suture retention.

For each lot, samples for both tensile and suture (1cm x 4cm) were tested. Samples were prepared by soaking them in deionized water for 30-35 minutes prior to testing. Tensile tests were performed by placing the samples between two grips such that a 25mm gap was tested. A 1N preload was applied and the test performed at 300mm/minute until failure-Both tensile load and elongation at failure were recorded. The suture testing was performed by threading a single 2.0 Prolene suture through one end of the test sample. The sample was placed in the grips at one end and the suture placed in the grips at the other such that a gap length of 60mm was achieved. A 1N, preload was applied and the test performed at 300mm/minute until failure. Young's Modulus was calculated from the tensile test results and sample measurements.

**Table 1: Average Mechanical Values for Samples**

| **Sample** | **Tensile** **(N)** | **Suture** **(N)** | **Elongation** **(%)** | **Young' s Modulus** **(MPa)** |
|---|---|---|---|---|
| 250 | 45.73 | 4.75 | 30.09 | 156.67 |
| 360 | 62.92 | 9.03 | 24.76 | 184.77 |
| 440 | 107.74 | 10.83 | 17.62 | 517.62 |

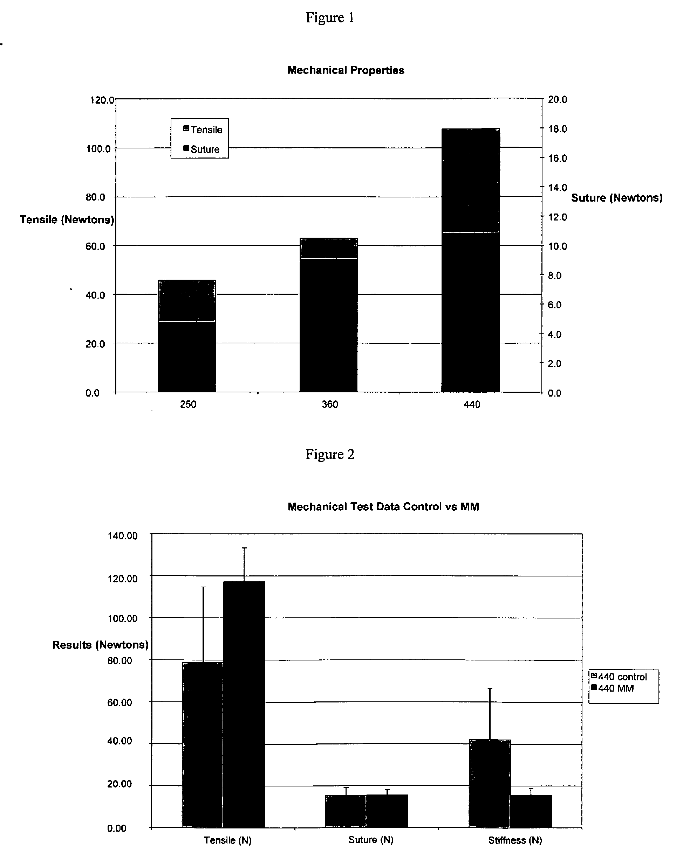

Table 1 and Figure 1 demonstrate the increased tensile strength, Young's Modulus and suture retention strength with increasing cellulose content. A decrease in elongation percent suggests that the material becomes stiffer with increasing cellulose.

### Example 7:

### Mechanical properties comparing pressed vs non-pressed implantable microbial cellulose

Materials were processed as in Examples 2 - 5. The mechanical properties of these implantable microbial cellulose forms were performed using a United Tensile Tester (Model SSTM-2kN). Testing included tensile strength, elongation, suture retention and fabric stiffness.

For each lot, samples for both tensile and suture (1cm x 4cm) and samples for stiffness (4cm x 5cm) were tested. Mechanical test samples were prepared by soaking them in deionized water for 30-35 minutes prior to testing. Tensile tests were performed by placing the samples between two grips such that a 25mm gap was tested. A IN preload was applied and the test performed at 300mm/minute until failure. Both tensile load and elongation at failure were recorded. The suture testing was performed by threading a single 2.0 Prolene suture through one end of the test sample. The sample was placed in the grips at one end and the suture placed in the grips at the other, such that a 60mm gap length was produced. A 1N preload was applied and the test performed at 300mm/minute until failure. Stiffness testing was performed by placing a 4cm x 5cm piece of test material onto a fabric stiffness testing rig. A 1.2cm diameter flat based probe was then lowered to the sample and the peak force needed to push the test material through a 2.5cm (OD), 2cm ID diameter hole with a 45-degree beveled edge was recorded.

**Table 2: Average Mechanical Values for Samples**

| **Sample** | **Process** | **Tensile** **(N)** | **Suture** **(N)** | **Elongation** **(%)** | **Stiffness** **(N)** |
|---|---|---|---|---|---|
| 360 | Control | 67.49 | 9.34 | 24.97 | 23.60 |
| 360P | Pressed | 79.30 | 8.77 | 20.27 | 6.09 |
| | | | | | |
| 440 | Control | 78.73 | 15.25 | 29.16 | 42.00 |
| 440P | Pressed | 117.21 | 15.42 | 18.34 | 15.58 |

Table 2 and Figure 2 demonstrate the increased tensile strength of the compressed samples over uncompressed controls, but no change in the suture retention strength.

### Example 8:

### Absorption Testing

Material in the 440 configurations was prepared as in Examples 3 (SCD Control) and Example 5 (SCD Pounded) as well as following US Patent '518 (Solvent Dehydrated). A fourth material was prepared as in Example 3, but was then pressed for 60 seconds under 80 PSI of pressure (SCD 60 sec Press). This resulted in an intermediate thickness material between SCD Control and SCD Pounded. Samples were either tested for tensile or suture strength after being hydrated for 30-35 minutes as in Example 7 or underwent absorption testing as follows. Samples measuring 4 x 5 cm were placed on top of a sponge that was soaked in a 0.9% saline solution and the testing chamber closed. Weights of the samples were taken prior to testing and at 24 hours after placement on the sponge.

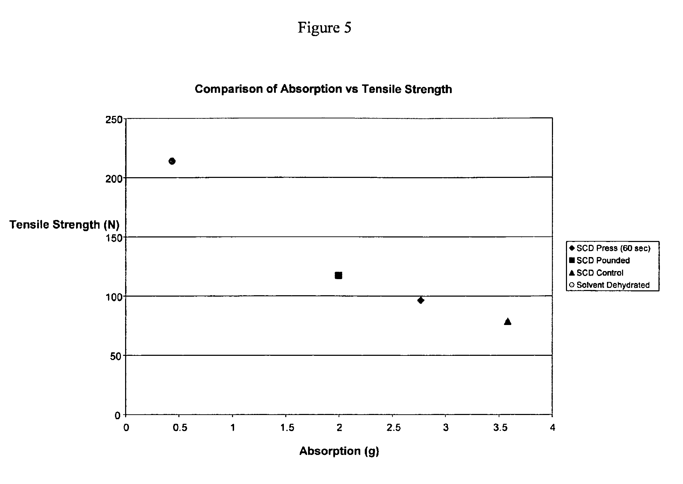

Figure 5 illustrates the comparison of absorption amount versus tensile strength for the various materials. Note that the stronger a material is in tension the less it absorbs. This demonstrates that the '518 material (solvent dehydrated) would not be useful as a delivery vehicle for a medically useful agent when compared to material processed using any of the SCD methods.

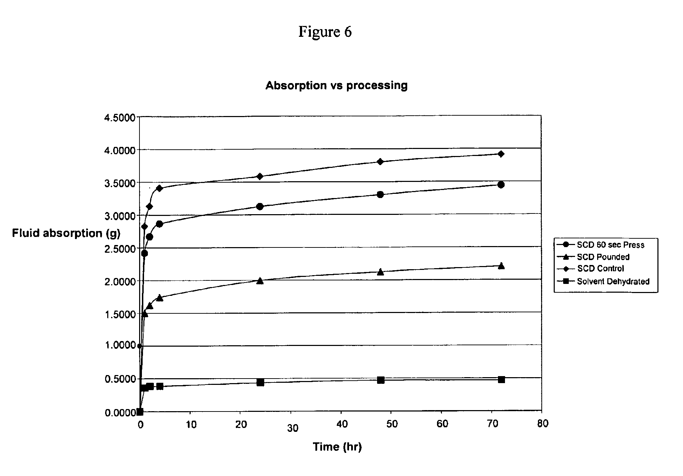

Figure 6 illustrates that even over time the '518 (solvent dehydrated) never reaches the level of absorption observed with materials prepared as described in the current invention.

### Example 9

### Physical characteristics of Implantable Microbial Cellulose material

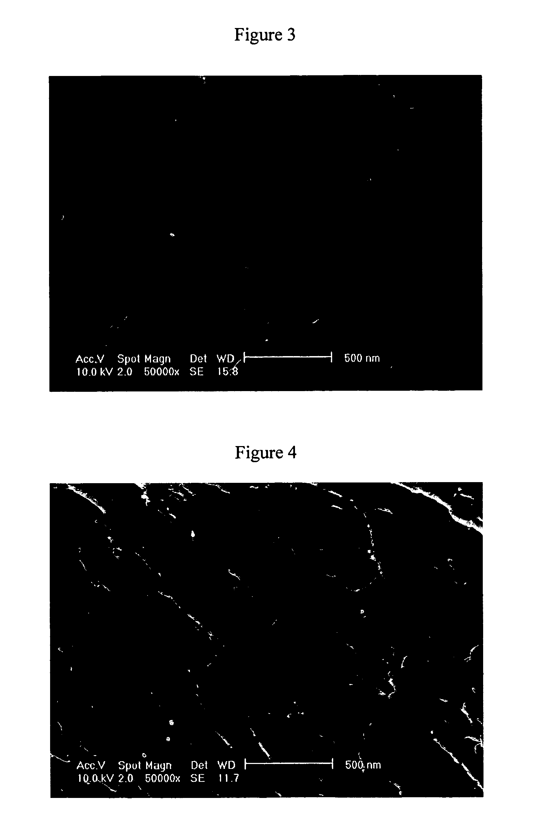

The physical characteristics of implantable microbial cellulose can be seen in Figures 3 and 4 that show an SEM image of the implantable microbial cellulose materials. Note the interconnected fibers in Figure 3 and the laminar structure in Figure 4.

### Example 10:

### Safety testing of Implantable Microbial Cellulose

Biocompatibility testing and implantation studies were conducted to assess the implantable microbial cellulose safety profile. A battery of in vitro and animal biocompatibility tests including cytotoxicity, sensitization, intracutaneous irritation, systemic toxicity and genotoxicity have been conducted on microbial cellulose, with the results indicating that the material is biocompatible. Muscle implantation studies in rabbits up to 24 weeks have been performed and the histological and gross necropsy results showed no significant tissue reaction, minimal cellular interaction, and very low adhesion to tissue. Table 3 lists the tests and the results.

**Table 3: Biocompatibility testing and results of implantable microbial cellulose**

| Test | Results |
|---|---|
| Cytotoxicity | Pass |
| Irritation | Pass |
| Acute Systemic Toxicity | Pass |
| Genotoxicity - Bacterial Reverse Mutations | Pass |
| Genotoxicity - In vitro Chromosomal Aberration | Pass |
| Genotoxicity - Mouse Bone Marrow Micronucleus | Pass |
| Sensitization | Pass |
| 4, 12, 18 and 26 week Rabbit Muscle Implantation | Pass |
| Hemolysis | Pass |
| Subchronic Toxicity | Pass |
| Chronic Toxicity | Pass |
| Endotoxin (pyrogen level) | Pass |

### Example 10:

### Comparison with existing implantable medical devices indicated for shoulder repair applications

The implantable microbial cellulose materials prepared in examples 1-3 were compared with existing medical devices used for shoulder repair. The suture retention properties of collagen-based products (both human- and animal-derived) and PTFE materials were compared to the implantable microbial cellulose. These were tested in the following in vitro model.

Chicken Achilles tendons were harvested from fresh legs and stored in isotonic saline before use. These specimens were approximately 5cm long, 1cm wide and 2mm thick. Test specimens were individually prepared and tested on an Instron Mini 44 machine.

The test fixture consisted of a 3-mm thick aluminum 'L' shaped plate with three 0.5mm holes (hole edges polished to prevent suture damage) spaced 2.5mm in a row on both the side and top

The top holes were used for pull-off testing and the side holes for shear testing. No.2 Mersilene^{™} suture was introduced from the back of the plate and through the tendon and graft (when present) and returned through the tendon maintaining either a 2.5mm or 5mm suture gap and tied on the back of the plate with a square knot. The plate was held by one grip of the Instron and the free end of the tendon passed through a small eye bolt held by the other grip and constrained with a hemostat. Tension was applied at 1.0cm/min until failure. Failure load and mechanism of failure were determined.

Tendon with no graft and suture gaps of 2.5mm and 5mm served as controls. Test materials included PTFE-Teflon fabric (Gore-Tex^{®} Soft Tissue Patch), human derived cross-linked collagen (GRAFTJACKET^{™}), bovine pericardium (Peri - Guard), and implantable cellulose material of Examples 1 and 2. Test units were one centimeter square patches with 5-mm suture spacing.

Twelve specimens were run for each test material and graft material. The two lowest failure load values were discarded (due to loosening of knot or tendon clamp) and the remaining ten averaged to determine failure strength. Averages were compared by unpaired Student's t-Tests.

Results of the tests are given in Table 4. The failure loads occurred when the repairs first began to displace and these loads gradually decreased as the suture/graft passes through the tendon. The implantable microbial cellulose and bovine pericardium grafts were significantly (p< 0.05) stronger than the non-augmented suture for both tests. All non-augmented specimens failed by the suture slicing along the tendon (tear-out). Only the human collagen graft failed in a similar manner. The other grafts failed by various amounts of cut-out and tear-out with the bovine pericardium failing mainly by tear-out. However in all of the cut-out failures, the suture did not cut through any of the grafts but pulled them into and usually through the tendon. The graft/suture subsequently sliced along the tendon. These cut-out failures generally resulted in higher strengths.

**Table 4 Strength of tendon augmentations in Newtons (SD).**

| Augmentation | Shear | Pull-off |
|---|---|---|
| None-2.5 mm suture | 23 (8) | 27 (9) |
| gap | 100%T | 100%T |
| None-5 mm suture | 35 (5) | 36 (8) |
| gap | 100%T | 100%T |
| PTFE | 39 (7) | 49 (8) |
| | 100%T | 40%T |
| Bovine pericardium | 56 (14) | 49 (19) |
| | 60%T | 80%T |
| Human collagen | 41 (19) | 34 (9) |
| | 100%T | 100%T |
| Implantable | 48 (24) | 35 (14) |
| Cellulose | 40%T | 20%T |

| | | |
|---|---|---|
| T = percentage of tests that failed by tear-out. | | |

As is apparent from the preceding description and examples, the present invention is directed to a class of implantable materials using microbial cellulose that can be used for medical applications and medical devices to repair and replace injured orthopedic soft tissue. The products maybe constructed in variety of forms (e.g. film, pad, hydrated, dry) and with varying physical and chemical properties. Additionally, the materials can be used in combination with other biomaterials such as collagen, proteins, and other bioactive agents to enhance its efficacy for a particular application. Many other variations and details of construction, composition and configuration will be apparent to those skilled in the art and such variations are contemplated within the scope of the present invention.

### Example 11:

Material was initially cleaned, whitened, and dried as in Example 3. It was further processed by subjecting it to perforation by placing it in a tissue 1/1 mesher. This made macroscopic holes in the material while maintaining strength in one dimension.
The invention in particular pertains to the following subject matter:
1. A microbial cellulose, obtainable by drying microbial cellulose and subsequently physically modifying the dried microbial cellulose.
2. The microbial cellulose according to item 1,
   wherein the drying is carried out by supercritical fluid drying.
3. The microbial cellulose according to item 1 or item 2, wherein the physical modification is carried out by pressure means.
4. The microbial cellulose according to one or more of the preceding items, wherein the microbial cellulose has a tensile strength of at least 80 N and an absorption capability of at least 1 g/20 cm².
5. A microbial cellulose having a tensile strength of at least 80 N and an absorption capability of at least 1 g / 20 cm².
6. The microbial cellulose according to one or more of the preceding items, wherein the microbial cellulose is produced from *Acetobacter xylinum.*
7. The microbial cellulose according to one or more of the preceding items, wherein the microbial cellulose is sterilized.
8. The microbial cellulose according to item 7, wherein the microbial cellulose is sterilized by ionizing irradiation, by steam and pressure, or by ethylene oxide.
9. An implantable composition comprising the microbial cellulose of one or more of the preceding items.
10. An implantable composition comprising a medically useful substance and microbial cellulose.
11. The composition according to item 10, wherein the medically useful substance is a growth factor or a drug.
12. Use of microbial cellulose for the manufacture of an implant material for the repair of replacement of soft tissue.
13. Use according to item 12, wherein the implant material is a tissue scaffold, such as a tissue scaffold suitable as a suture buttress composition, such as a composition for anchoring soft tissue to bone, or a shoulder repair composition such as a rotator cuff composition or a labrum repair composition.
14. A method for producing a microbial cellulose, comprising the steps
   a) drying the microbial cellulose using supercritical fluid drying,
   b) pressing the dried cellulose.
15. A kit comprised of
   a) a microbial cellulose device according to item 1, and
   b) a sterilizable closable container.

## Claims

1. A microbial cellulose, obtainable by drying microbial cellulose and subsequently pressing the dried microbial cellulose.

2. The microbial cellulose according to claim 1, wherein the drying is carried out by supercritical fluid drying.

3. The microbial cellulose according to one or more of the preceding claims,
wherein the microbial cellulose has a tensile strength of at least 80 N and an absorption capability of at least 1 g/20 cm².

4. A microbial cellulose having a tensile strength of at least 80 N and an absorption capability of at least 1 g / 20 cm².

5. The microbial cellulose according to one or more of the preceding claims,
wherein the microbial cellulose is produced from *Acetobacter xylinum.*

6. The microbial cellulose according to one or more of the preceding claims, wherein the microbial cellulose is sterilized.

7. The microbial cellulose according to claim 6, wherein the microbial cellulose is sterilized by ionizing irradiation, by steam and pressure, or by ethylene oxide.

8. An implantable composition comprising the microbial cellulose of one or more of the preceding claims.

9. The implantable composition according to claim 8, comprising a medically useful substance.

10. The implantable composition according to claim 9,
wherein the medically useful substance is a growth factor or a drug.

11. Use of microbial cellulose for the manufacture of an implant material for the repair of replacement of soft tissue, the implant material contains microbial cellulose and has a tensile strength of at least 80 N.

12. Use according to claim 11,
wherein the implant material is a tissue scaffold, such as a tissue scaffold suitable as a suture buttress composition, such as a composition for anchoring soft tissue to bone, or a shoulder repair composition such as a rotator cuff composition or a labrum repair composition.

13. A kit comprising
a) a microbial cellulose device according to one or more of claims 1 to 7; and
b) a sterilizable closable container.
